# EUROPÄISCHE PATENTSCHRIFT

(11) **EP 0 918 489 B1**
(45) Veröffentlichungstag und Bekanntmachung des Hinweises auf die Patenterteilung: **13.02.2002**
(21) Anmeldenummer: 97935464.4
(22) Anmeldetag: 22.07.1997
(51) Int. Cl.: A61B 17/34

(54) **TROKARHÜLSE FÜR DIE ENDOSKOPIE**
TROCAR SHEATH FOR ENDOSCOPIC USE
GAINE DE TROCART S'UTILISANT POUR L'ENDOSCOPIE

(30) Priorität: 22.07.1996 DE 19629537
(43) Veröffentlichungstag der Anmeldung: 02.06.1999
(73) Patentinhaber: Karl Storz GmbH & Co. KG, 78532 Tuttlingen (DE)
(72) Erfinder: MANHES, Hubert, F-03200 Vichy (FR)
(74) Vertreter: Münich, Wilhelm, Dr.
(86) Internationale Anmeldenummer: DE9701552
(87) Internationale Veröffentlichungsnummer: WO9803121

(56) Entgegenhaltungen:
- EP-A- 0 577 400
- EP-A- 0 614 646
- WO-A-97/33520
- DE-A- 4 238 596
- US-A- 5 263 937
- US-A- 5 279 564
- US-A- 5 279 575
- US-A- 5 354 302

## Beschreibung

### Technisches Gebiet

Die Erfindung bezieht sich auf eine Trokarhülse für die Endoskopiemit einem länglichen Teil, der wenigstens einen Kanal auf-weist, in den ein Instrument einsetzbar ist, und mit distalseitig beweglichen, schwenkbaren Teilen.

### Stand der Technik

Typische Trokarhülsen weisen einen zylindrischen Abschnitt mit wenigstens einem Kanal auf, in den ein Instrument, wie ein Trokardorn, ein Endoskop, eine Schere o. dgl. einsetzbar ist. Der Großteil der derzeit auf dem Markt befindlichen Trokarhülsen hat eine Länge, die wesentlich größer ist als die Dicke der Wandung der Körperhöhle, wie beispielsweise der Bauchdecke ist, durch die der Trokar bzw. die Trokarhülse in das Körperinnere eingesetzt ist. Der Grund hierfür ist unter anderem, daß ein zu kurzer Trokar in der Praxis weder gut gegriffen noch beim Einführen geführt werden kann. Damit beschränkt der zylindrische Rohrabschnitt der Trokarhülse den Bereich der Körperhöhle, der mit einem flexiblen Instrument oder mit einem Instrument, dessen Durchmesser kleiner als der des Trokarkanals ist, zugänglich ist.

Dieser Nachteil ist auch bei der aus der DE 43 07 228 A1, von der unter anderem bei der Formulierung des Oberbegriffs des Patentanspruchs 1 ausgegangen wird, bekannten Trokarhülse vorhanden:

Diese Trokarhülse besteht aus zwei Hülsen, von denen die äußere mit beweglichen schwenkbaren Teilen, wie Segmenten, Plättchen, Stangen etc. versehen ist. Die innere Hülse ist dagegen in Art bekannter Trokare starr ausgebildet.

Entsprechendes gilt für die aus der DE 82 16 373 U1 bekannten Trokarhülse, die spreitzbare, aber keine schwenkbaren Endabschnitte aufweist.

### Darstellung der Erfindung

Aufgabe der Erfindung ist es, eine Trokarhülse anzugeben, die in eine Körperhöhle beispielsweise durch Durchstoßen der Bauchdecke eingesetzt werden kann, ohne dass ein Trokardorn verwendet werden müsste.

Der Erfindung liegt weiter die Aufgabe zugrunde, eine Trokarhülse anzugeben, die während des Einführvorgangs in eine Körperhöhle eine so große Länge hat, dass sie ergonomisch und sicher gehandhabt werden kann, und die dennoch nach dem Einführen in die Körperhöhle nicht das Arbeiten in der Körperhöhle dadurch behindert, dass sie zu weit in die Körperhöhle ragt.

Aus der US 5,279,575 ist eine Trokarhülse bekannt, deren distaler Bereich von zwei Längsabschnitten oder Flügeln gebildet wird, die derart nach vorn geklappt werden können, dass sie eine rund ausgebildete Spitze bilden. Die runde Ausführung der Spitzen ermöglicht nicht das Durchstoßen der Bauchdecke ohne zusätzlichen Trokardorn oder ein zusätzliches dafür geeignetes Instrument. Zur Durchdringung der Haut muss der Operateur einen kleinen Einschnitt vornehmen, bevor der Trokardorn eingeführt werden kann.
Der US 5,279,575 ist auch nicht ein Mechanismus zu entnehmen, der ein gezieltes Verschwenken der einzelnen distalseitigen Längsabschnitte erlaubt. Von dieser Lösung wurde bei der Bildung des Oberbegriffs des Patentanspruchs 1 ebenfalls ausgegangen.

Eine erfindungsgemäße Lösung dieser Aufgaben ist im Patentanspruch 1 angegeben.

Vorteilhafte Weiterbildungen des Gegenstands des Patentanspruchs 1 sind in den Ansprüchen 2 bis 8 angegeben.

Der erfindungsgemäße Grundgedanke, den distalen Abschnitt der Trokarhülse in Längsrichtung zu teilen, und die einzelnen Längsabschnitt schwenkbar auszugestalten, kann nicht nur dazu benutzt werden, den erzielbaren "Öffnungskegel" zu vergrößern, sondern insbesondere dazu, die Längsabschnitte derart nach vorne klappbar auszubilden, dass sie eine Spitze bilden (Anspruch 1). Dabei ist es gegebenenfalls erforderlich, die einzelnen Abschnitte mit einer Schneide bzw. einer Spitze zu versehen, so dass auch die kegelförmig nach vorne geklappten Längsabschnitte eine gemeinsame Spitze haben. Damit ist es möglich, auf den spitzen Trokardorn zu verzichten, und die Trokarhülse aufgrund ihrer Spitze ohne zusätzlichen Dorn direkt durch die Körperwandung zu "stoßen".

Allen Lösungsformen liegt als gemeinsamer Grundgedanke zugrunde, dass um eine zur Längsachse der Trokarhülse senkrechte Achse schwenkbare Längsabschnitte den distalen Bereich der Trokarhülse bilden. Schwenkt man die Längsabschnitte nach dem Einführen der Trokarhülse in die Körperhöhle nach außen, wird die effektive Länge der Trokarhülse verkürzt. Damit kann ein flexibles oder abwinkelbares Instrument einen größeren Teil der Körperhöhle erreichen, als bei einer herkömmlichen Trokarhülse, die vergleichsweise weit in die Körperhöhle ragt. Dennoch ist die Handhabbarkeit der Trokarhülse beim Durchstoßen beispielsweise der Bauchdecke nicht eingeschränkt, da der Trokar bzw. die Trokarhülse eine Länge hat, die ein bequemes Greifen erlaubt.

Wie bereits ausgeführt, ist bei der im Anspruch 2 angegebenen Lösung die Trokarhülse in ihrem distalen Endbereich in Längsrichtung geteilt, so dass sie aus mehreren Längsabschnitten besteht. Die einzelnen Längsabschnitte sind nach dem Einsetzen der Trokarhülse nach außen in Richtung auf das proximale Ende hin umklappbar. Damit kann der Öffnungskegel, den der oder die Kanäle der Trokarhülse für flexible bzw. abwinkelbare Instrumente freigeben, gegenüber herkömmlichen Trokarhülsen wesentlich vergrößert werden. Auch dann, wenn Instrumente mit einem deutlich geringeren Durchmesser als der Kanaldurchmesser "schräg" durch
den Kanal geführt werden, kann ein wesentlich größerer Raum "bearbeitet" werden.

Die nach außen schwenkbaren Längsabschnitte können nicht nur dazu benutzt werden, den für Instrumente zugänglichen Raum der Körperhöhle zu vergrößern, sondern auch dazu, die Trokarhülse an der Wandung der Körperhöhle, also beispielsweise der Bauchdecke zu befestigen bzw. fixieren:

Hierzu sind die Längsabschnitte an die Innenwandung der Körperhöhle anlegbar, in die die Trokarhülse eingesetzt ist. Dabei ist es von besonderem Vorteil, wenn die Längsabschnitte in Art von Flügeln ausgebildet sind, da dann die Längsabschnitte mit einer großen Fläche an der Körperwandung anliegen.

Bei einer weiteren Ausgestaltung ist am proximalen Ende der Trokarhülse ein Flansch vorgesehen, mit dem die Trokarhülse an der Außenwandung der Körperhöhle anliegt. Damit kann die Trokarhülse in einer "Art" Zangengriff an der Körperwandung befestigt werden, wobei sie von innen durch die Flügel bzw. die nach außen geklappten Längsabschnitte abgestützt wird.

Hierzu ist es bevorzugt, wenn der Flansch in Längsrichtung verstellbar ist, da dann die Körperwandung zwischen den Flansch und die aufklappbaren Längsabschnitte "eingespannt" werden kann.

Der Mechanismus, mit dem die Längsabschnitte nach innen und/oder nach außen geklappt werden, kann auf die verschiedensten Arten ausgeführt werden:

So ist es beispielsweise möglich, Übertragungselemente, wie Seilzüge, Stangen etc. vorzusehen, mit denen der Klappvorgang bewerkstelligt wird. Eine besonders einfache Lösung ist im Anspruch 6 angegeben: Gemäß dieser Lösung weist der Mechanismus Federelemente auf, die die einzelnen Längsabschnitte in Richtung auf das proximale Ende beaufschlagen. Dabei sind die Längsabschnitte in ihrer "Ruhestellung" nach innen geklappt, so daß sie die im Anspruch 2 angegebene Spitze bilden. Nach dem Durchstoßen der Körperwandung wird ein Instrument, beispielsweise ein Endoskop eingeführt. Hierdurch werden die Längsabschnitte zwangsweise nach außen geschwenkt. Sobald die Stellung der Längsabschnitte einen bestimmten Winkel überschreitet, klappen die Längsabschnitte aufgrund der Federwirkung nach außen um, und können dabei insbesondere an der Innenseite der Körperhöhle, also beispielsweise an der Innenseite der Bauchdecke zur Anlage kommen.

Zum Herausziehen der Trokarhülse aus der Körperhöhle werden durch die Anlage der Längsabschnitte an der Innennseite der Körperhöhle diese nach vorne geschwenkt, so daß die Trokarhülse ohne Probleme entnommen werden kann. Dabei kann das Instrument in dem Kanal der Trokarhülse verbleiben.

Der erfindungsgemäße Trokar kann praktisch auf sämtlichen Gebieten der Endoskopie, wie beispielsweise der Zölioskopie oder der Laparoskopie, aber auch bei technischen Anwendungen eingesetzt werden.

### Kurze Beschreibung der Zeichnung

Die Erfindung wird nachstehend ohne Beschränkung des allgemeinen Erfindungsgedankens anhand von Ausführungsbeispielen unter Bezugnahme auf die Zeichnung exemplarisch beschrieben, auf die im übrigen bezüglich der Offenbarung aller im Text nicht näher erläuterten erfindungsgemäßen Einzelheiten ausdrücklich verwiesen wird. Es zeigen:
- Fig. 1: einen Längsschnitt durch eine erfindungsgemäße Trokarhülse, und
- Fig. 2: eine herkömmliche Trokarhülse.

### Darstellung eines Ausführungsbeispiels

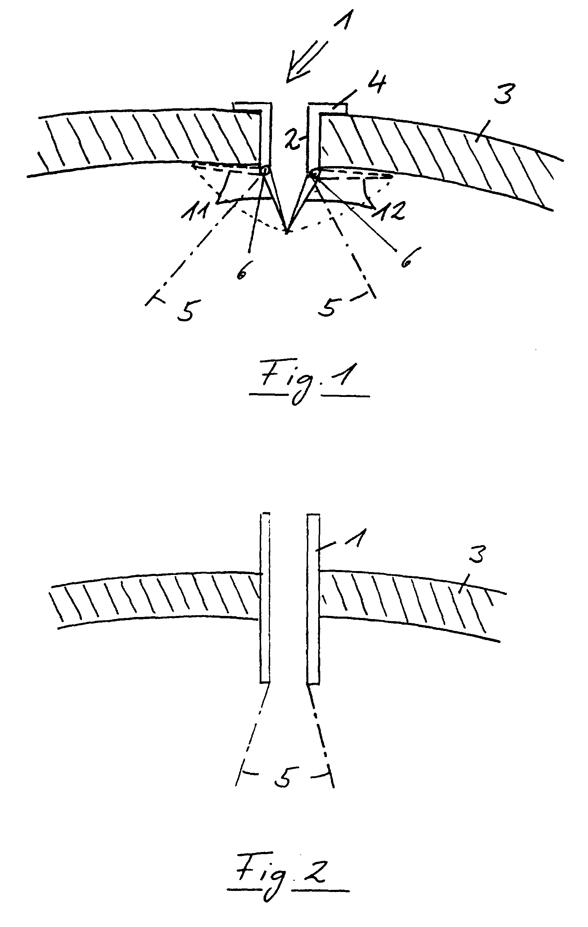

Fig. 1 zeigt eine erfindungsgemäße Trokarhülse 1. Die Trokarhülse ist in ihrem distalen Endbereich in Längsrichtung geteilt, so daß sie aus mehreren - bei dem gezeigten Ausführungsbeispiel vier Längsabschnitten - besteht, von denen in Fig. 1 nur die Längsabschnitte 11 und 12 dargestellt sind. Möglich sind aber auch zwei, drei, oder mehr also beispielsweise fünf Längsabschnitte.

Die Längsabschnitte 11,12 sind an einem zylindrischen Mittelabschnitt 2 der Trokarhülse 1 mittels Gelenke 6 schwenkbar angelenkt und über geeignete Betätigungselemente vom proximalen Ende aus oder durch eine Federwirkung schwenkbar.

Dabei ist es beispielsweise möglich, daß Federelemente in den Gelenken 6 auf die einzelnen Längsabschnitte 11,12 in Richtung auf das proximale Ende beaufschlagen. Dabei sind die Längsabschnitte normalerweise nach innen geklappt, so daß sie die mit ausgezogenen Linien dargestellte Spitze bilden. Damit ist es beim Einführen des Trokars 1 möglich, zum Durchstoßen der Körperwandung, wie der Bauchdecke 3, auf einen Trokardorn zu verzichten.

Nach dem Einführen der Trokarhülse 1 werden die einzelnen Längsabschnitte (11,12) nach außen umgeklappt, bis sie an der Innenwand der Bauchdecke 3 anliegen. Dies ist in Fig. 1 gestrichelt dargestellt. Die Schwenkbewegung ist gestrichelt symbolisiert.

Im Falle der Verwendung von federbeaufschlagten Gelenken 6 kann beispielsweise nach dem Durchstoßen der Körperwandung ein nicht dargestelltes Instrument, wie ein Endoskop eingeführt werden. Hierdurch werden die Längsabschnitte zwangsweise nach außen geschwenkt. Sobald die Stellung der Längsabschnitte einen bestimmten Winkel überschreitet, klappen die Längsabschnitte aufgrund der Federwirkung nach außen um, und kommen damit an der Innenseite der Körperhöhle zur Anlage.

Am proximalen Ende der Trokarhülse 1 ist ein Flansch 4 vorgesehen ist, mit dem die Trokarhülse 1 an der Außenwand der Bauchdecke 3 anliegt, so daß der Trokar dadurch fixiert wird, daß die Bauchdecke 3 zwischen Flansch 4 und den Längsabschnitten 11, 12 "eingespannt" ist. Hierzu kann der Flansch 4 in Längsrichtung längs des Mittelabschnitts 2 verstellbar sein.

Zum Herausziehen der Trokarhülse aus der Körperhöhle werden durch die Anlage der Längsabschnitte 11,12 an der Innennseite der Bauchdecke 3 die Längsabschnitte nach vorne geschwenkt, so daß die Trokarhülse ohne Probleme entnommen werden kann. Dabei kann das Instrument in dem Kanal der Trokarhülse verbleiben.

Strichpunktiert ist der für ein - nicht dargestelltes-Instrument zugängliche Raum 5 dargestellt.

Fig. 2 zeigt zum Vergleich den mit einem herkömmlichen-lediglich aus einem langen Rohrabschnitt 2' bestehenden-Trokar 1 zugänglichen Raum 5. Wie einem Vergleich der Figuren zu entnehmen ist, ist mit dem erfindungsgemäßen Trokar ein wesentlich größerer Raum zugänglich. Darüberhinaus kann auf einen gesonderten Trokardorn verzichtet werden.

Vorstehend ist die Erfindung ohne Beschränkung der allgemeinen Anwendbarkeit auf allen Gebieten der Endoskopie beschrieben worden. Anstelle der beschriebenen Ausführungsform können selbstverständlich auch die verschiedensten anderen Ausführungsformen verwendet werden:

So ist es möglich, anstelle von federbeaufschlagten Gelenken 6 die Längsabschnitte 11,12 durch Seilzüge, Hebelgelenke oder durch einen in den Trokarkanal einführbaren Bedienungsmechanismus zu betätigen.

## Patentansprüche

1. Trokarhülse für die Endoskopie, mit
- einem länglichen Teil, der wenigstens einen Kanal aufweist, in den ein Instrument einsetzbar ist,
- distalseitig beweglichen, schwenkbaren Teilen, und
- mit einem distalen Bereich, der von mehreren Längsabschnitten (11,12) gebildet wird, die an dem proximalen Teil (2) der Trokarhülse angelenkt sind,
**dadurch gekennzeichnet, dass** die Längsabschnitte (11, 12), die den distalen Bereich der Trokarhülse bilden, in Richtung zum distalen Ende um eine zur Längsachse der Trokarhülse senkrechte Achse nach vorne geklappt werden können, und dass die Längsabschnitte (11, 12) derart ausgebildet sind, dass sie dabei eine Spitze bilden, die das Durchstoßen der Körperdecke (3) auch ohne zusätzlichen Trokardorn erlaubt.

2. Trokarhülse nach Anspruch 1,
**dadurch gekennzeichnet, dass** ein Mechanismus vorgesehen ist, der die einzelnen Längsabschnitte (11,12) um jeweils eigene zur Längsachse der Trokarhülse senkrechte Achsen verschwenkt.

3. Trokarhülse nach Anspruch 1 oder 2,
**dadurch gekennzeichnet, dass** nach dem Einsetzen der Trokarhülse (1) in eine Körperhöhle die Längsabschnitte (11, 12) nach außen in Richtung auf das proximale Ende hin umgeklappt werden können.

4. Trokarhülse nach Anspruch 1,
**dadurch gekennzeichnet, dass** die Längsabschnitte (11, 12) an die Innenwandung (3) der Körperhöhle anlegbar sind, in die die Trokarhülse eingesetzt ist.

5. Trokarhülse nach einem der Ansprüche 1 bis 4,
**dadurch gekennzeichnet, dass** die Längsabschnitte (11, 12) in Art von Flügeln ausgebildet sind.

6. Trokarhülse nach einem der Ansprüche 2 bis 5,
**dadurch gekennzeichnet, dass** der Mechanismus Federelemente aufweist, die die einzelnen Längsabschnitte in Richtung auf das proximale Ende beaufschlagen.

7. Trokarhülse nach einem der Ansprüche 1 bis 6,
**dadurch gekennzeichnet, dass** am proximalen Ende der Trokarhülse (1) ein Flansch (4) vorgesehen ist, mit dem die Trokarhülse (1) an der Außenwandung der Körperhöhle (3) anlegbar ist.

8. Trokarhülse nach Anspruch 7,
**dadurch gekennzeichnet, dass** der Flansch (4) in Richtung der Längsachse des Trokars (1) verstellbar ist.

## Claims

1. Trocar sheath for use in endoscopy, comprising
- an elongate part including at least one duct for introduction of an instrument,
- mobile pivotable parts on the distal side, and
- a distal region formed by several longitudinal sections (11, 12) articulated on the proximal part (2) of said trocar sheath,
**characterised in that** said longitudinal sections (11, 12) constituting the distal region of said trocar sheath are adapted to be folded in the forward direction towards the distal end about an axis orthogonal on the longitudinal axis of the trocar sheath, and that said longitudinal sections (11, 12) are so configured that they form a tip permitting the piercing of the body wall (3) also without an additional trocar mandrel.

2. Trocar sheath according to Claim 1,
**characterised in that** a mechanism is provided for pivoting the individual longitudinal sections (11, 12) about respective separate axes orthogonal on the longitudinal axis of the trocar sheath.

3. Trocar sheath according to Claim 1 or 2,
**characterised in that** after insertion of the trocar sheath (1) into a body cavity the longitudinal sections (11, 12) may be folded outwardly I a direction towards the proximal end.

4. Trocar sheath according to Claim 1,
**characterised in that** said longitudinal sections (11, 12) are adapted to be positioned against the inner wall (3) of said body cavity into which the trocar sheath is inserted.

5. Trocar sheath according to any of the Claims 1 to 4,
**characterised in that** said longitudinal sections (11, 12) are configured in the manner of wings.

6. Trocar sheath according to any of the Claims 2 to 5,
**characterised in that** said mechanism comprises spring elements acting upon the individual longitudinal sections to bias them in a direction towards the proximal end.

7. Trocar sheath according to any of the Claims 1 to 6,
**characterised in that** a flange (4) is provided on the proximal end of the trocar sheath, by which the trocar sheath (1) can be caused to bear against the outer wall of the body cavity (3).

8. Trocar sheath according to Claim 7,
**characterised in that** said flange (4) is adjustable along the direction of the trocar (1).

## Revendications

1. Gaine de trocart s'utilisant pour l'endoscopie, comprenant
- une partie allongée renfermant au moins un canal pour l'introduction d'un instrument,
- des éléments pivotables mobiles du côté distal, et
- une zone distale formée par une pluralité segments longitudinaux (11, 12) articulés à la partie proximale (2) de la gaine de trocart,
**caractérisée en ce que** lesdits segments longitudinaux (11, 12), qui constituent la région distale de la gaine de trocart, sont aptes à être pliés à l'avant vers l'extrémité distale autour d'un axe orthogonal sur l'axe longitudinal de la gaine de trocart, et **en ce que** lesdits segments longitudinaux (11, 12) sont configurés de façon, qu'ils forment une pointe, qui permet le perçage de la paroi corporelle (3) aussi sans un goujon de trocart supplémentaire.

2. Gaine de trocart selon la revendication 1,
**caractérisée en ce qu'**un mécanisme est disposé à pivoter les segments longitudinaux (11, 12) individuels autour des axes séparés respectifs, qui sont orthogonaux sur l'axe longitudinal de la gaine de trocart.

3. Gaine de trocart selon la revendication 1 ou 2,
**caractérisée en ce que** suivant l'insertion de la gaine de trocart (1) dans une cavité corporelle desdits segments longitudinaux (11, 12) peuvent être pliés à l'extérieur en un sens vers l'extrémité proximale.

4. Gaine de trocart selon la revendication 1,
**caractérisée en ce que** lesdits segments longitudinaux (11, 12) sont aptes à être adhérés contre la paroi intérieure (3) de ladite cavité corporelle, dans laquelle la gaine de trocart est insérée.

5. Gaine de trocart selon une quelconque des revendications 1 à 4,
**caractérisée en ce que** lesdits segments longitudinaux (11, 12) sont configurés de la manière des ailettes.

6. Gaine de trocart selon une quelconque des revendications 2 à 5,
**caractérisée en ce que** ledit mécanisme comprend des éléments à ressort, qui agissent sur les segments longitudinaux individuels afin de les mettre en précontrainte en un sens vers l'extrémité proximale.

7. Gaine de trocart selon une quelconque des revendications 1 à 6,
**caractérisée en ce qu'**une collerette (4) est disposée sur l'extrémité proximale de la gaine de trocart, moyennant laquelle on peut causer la gaine de trocart (1) à porter contre la paroi extérieure de la cavité corporelle (3).

8. Gaine de trocart selon la revendication 7,
**caractérisée en ce que** ladite collerette (4) est ajustable le long de l'extension du trocart (1).
